(19) Europäisches Patentamt
European Patent Office
Office européen des brevets

(11) EP 1 093 806 B1

(12) **FASCICULE DE BREVET EUROPEEN**

(45) Date de publication et mention de la délivrance du brevet:
**28.12.2005 Bulletin 2005/52**

(51) Int Cl.[7]: **A61K 7/50**, A61K 7/02, A61K 7/075, A61K 7/06

(21) Numéro de dépôt: **00402724.9**

(22) Date de dépôt: **04.10.2000**

(54) **Compositions cosmétiques contenant un copolymère vinyldiméthicone/diméthicone et une silicone et leurs utilisations**

Kosmetische Zusammensetzungen enthaltend ein Vinyldimethicone/Dimethicon-Copolymer und ein Silicon und deren Verwendung

Cosmetic compositions containing a vinyldimethicone/dimethicone copolymer and a silicone and their use

(84) Etats contractants désignés:
**AT BE CH CY DE DK ES FI FR GB GR IE IT LI LU MC NL PT SE**

(30) Priorité: **20.10.1999 FR 9913096**

(43) Date de publication de la demande:
**25.04.2001 Bulletin 2001/17**

(73) Titulaire: **L'OREAL**
**75008 Paris (FR)**

(72) Inventeurs:
- **Decoster, Sandrine**
**95210 Saint Gratien (FR)**
- **Douin, Véronique**
**75017 Paris (FR)**
- **Bailly, Virginie**
**92110 Clichy (FR)**

(74) Mandataire: **Le Blainvaux Bellegarde, Françoise**
**L'OREAL - D.I.P.I.**
**25-29 Quai Aulagnier**
**92600 Asnières (FR)**

(56) Documents cités:
**EP-A- 0 874 017**
**US-A- 5 360 851**
**US-A- 5 667 771**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 1 093 806 B1

## Description

La présente invention concerne de nouvelles compositions cosmétiques comprenant dans un milieu cosmétiquement acceptable au moins une silicone et au moins un copolymère dimethicone à insaturation éthyléniqueldimethicone.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des polymères cationiques ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légéreté du cheveu), un manque de lissage (cheveu non homogène de la racine à la pointe).

En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

US 5,667,71 décrit des compositions capillaires comprenant un copolymère silicone et un solvant volatile. Les exemples divulguent l'addition de diméthicone.

EP 0874 017 décrit un procédé pour fabriquer des émulsions de silicone particulières.

En résumé, il s'avère que les compositions cosmétiques actuelles contenant des silicones, ne donnent pas complètement satisfaction.

La demanderesse a maintenant découvert que l'association d'au moins un copolymère siliconé de viscosité comprise entre $10^6$ et $100.10^6$ cP avec des silicones permet de remédier à ces inconvénients.

Ainsi, à la suite d'importantes recherches menées sur la question, il a maintenant été trouvé par la Demanderesse qu'en introduisant au moins un copolymère siliconé particulier de viscosité comprise entre $10^6$ et $100.10^6$ cP dans les compositions en particulier capillaires de l'art antérieur à base de silicones, il est possible de limiter, voire supprimer, les problèmes généralement liés à l'emploi de telles compositions, à savoir en particulier l'alourdissement (toucher chargé lors d'applications répétées), le manque de lissage et de douceur des cheveux, tout en conservant les autres propriétés cosmétiques avantageuses qui sont attachés aux compositions à base d'agents conditionneurs.

Par ailleurs, les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable, au moins un copolymère siliconé tel que défini ci-dessous de viscosité comprise entre $10^6$ et $100.10^6$ cP et au moins une silicone.

Un autre objet de l'invention concerne l'utilisation d'au moins un copolymère siliconé tel que défini ci-dessous de viscosité comprise entre $10^6$ et $100.10^6$ cP dans, ou pour la fabrication d'une composition cosmétique comprenant une silicone.

Les différents objets de l'invention vont maintenant être détaillés. L'ensemble des significations et définitions des composés utilisés dans la présente invention données ci-dessous sont valables pour l'ensemble des objets de l'invention.

Le copolymère siliconé résulte de la réaction d'addition, en présence d'un catalyseur, d'au moins

- (a) un polysiloxane de formule (I) :

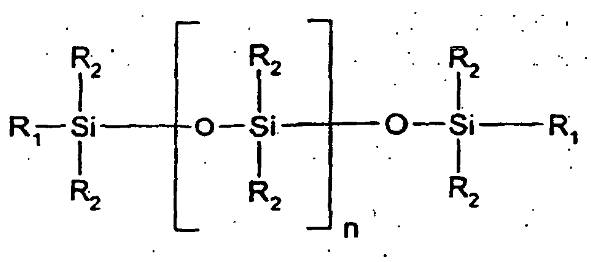

(I)

dans laquelle :

R1 désigne un groupement susceptible de réagir par réaction d'addition de chaîne tel que par exemple un atome d'hydrogène, un groupement aliphatique ayant une insaturation éthylénique notamment vinyl, allyl ou héxényl,

Les groupements R2 de la formule (I) peuvent représenter notamment des groupements alkyle, cycloalkyle, aryle, alkylaryle ou hydroxyle, et peuvent comporter en outre, des groupements fonctionnels tels qu'éthers, amines, carboxyles, hydroxyles, thiols, esters, sulfonates, sulfates.

Les groupements alkyle ont par exemple 1 à 20 atomes de carbone ; les groupements cycloalkyle ont par exemple 5 ou 6 atomes de carbone ; les groupements aryle sont notamment des groupements phényle ; les groupements alkylaryle peuvent avoir de 7 à 20 atomes de carbone.

Plus particulièrement R2 désigne méthyle.

n est un entier tel que le polysiloxane de formule (I) ait de préférence une viscosité cinématique comprise entre 1 et $1.10^6$ $mm^2/s$..n varie notamment de 5 à 5000.

- (b) et d'au moins un composé siliconé comprenant au moins un et au plus deux groupements susceptibles de réagir avec les groupements R1 du polysiloxane (a),

l'un au moins des composés de type (a) ou (b) contient un groupe aliphatique ayant une insaturation éthylénique.

Les composés de type (b) sont un autre polysiloxane de type (a) dans lequel les groupements R1 du polysiloxane (b) sont susceptibles de réagir avec les groupement R1 du polysiloxane (a).

De préférence, les copolymères siliconés sont notamment obtenus par réaction d'addition, en présence d'un catalyseur d'hydrosilylation (par exemple un catalyseur au platine), d'au moins :

- (a) un alpha,oméga-di vinyl polydiméthylsiloxane, et
- (b) d'un alpha,oméga-di hydrogéno polydiméthylsiloxane.

Le copolymère a généralement une viscosité dynamique, mesurée à la température d'environ 25°C et au taux de cisaillement de 0,01 Hz pour une contrainte de 1500 Pa, comprise entre $10^6$ et $100.10^6$ cP et de préférence comprise entre $5.10^6$ cP et $30.10^6$ cP.

Toutes les mesures de viscosités dynamiques données dans la présente demande ont été effectuées à une température d'environ 25°C, sur un Carri-Med CSL2-500.

La viscosité cinématique est par exemple mesurée à 25°C selon la norme ASTM 445 Appendice C.

Les copolymères siliconés selon l'invention sont essentiellement non réticulés.

Le copolymère siliconé présent dans la composition selon l'invention peut se présenter sous la forme d'émulsion aqueuse.

Par émulsion aqueuse, on entend une émulsion de type huile-dans-eau dans laquelle le copolymère siliconé est dispersé sous forme de particules ou de gouttelettes dans la phase aqueuse formant la phase continue de l'émulsion.

Cette émulsion peut être stabilisée par un système émulsionnant usuel.

Cette émulsion de silicone peut avoir une taille de gouttelettes ou de particules de silicone allant de 10 nm à 50 µm, et de préférence de 0,3 µm à 20 µm.

La taille des particules est mesurée par granulométrie laser.

Le système émulsionnant comprend des tensioactifs employés habituellement dans les émulsions de silicone. Ces tensioactifs peuvent être non-ioniques, cationiques, anioniques ou amphotères ou leurs mélanges tels que ceux décrits ci-dessous.

Le système émulsionnant représente de 0,5 % à 10 % en poids par rapport au poids total de l'émulsion.

La synthèse de ces émulsions de silicones est notamment décrite dans la demande EP-A-874017.

De telles émulsions sont notamment commercialisées sous la dénomination DC2-1997 cationic emulsion par la société DOW CORNING. Cette émulsion comprend un copolymère $\alpha,\bar{\omega}$-divinyl diméthicone/$\alpha,\bar{\omega}$-dihydrogénodiméthicone ayant une viscosité dynamique d'environ $15.10^6$ cP, un émulsionnant de type cationique tel que le chlorure de cétyltriméthylammonium, un stabilisant de type hydroxyéthylcellulose et de l'eau.

Le copolymère siliconé est utilisé de préférence en une quantité comprise entre 0,05 et 10% en poids par rapport au poids total de la composition. Plus préférentiellement, cette quantité est comprise entre 0,1 et 5% en poids par rapport au poids total de la composition.

Les silicones différentes du copolymère siliconé utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi:

(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.

On peut également citer les cyclocopolymères du type diméthylsiloxanesl méthylakylsiloxane, tel que la "SILICONE VOLATILE FZ 3109" commercialisée par la société UNION CARBIDE, de structure chimique :

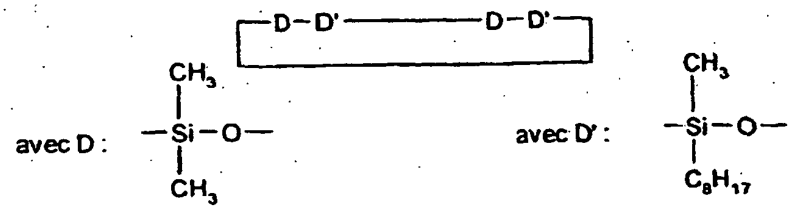

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;

(ii) les silicones volatiles linéaires ayant 2 à 9 atomes de silicium et possédant une viscosité inférieure ou égale à $5.10^{-6} m^2/s$ à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de $5.10^{-6}$ à 2,5 $m^2/s$ à 25°C et de préférence $1.10^{-5}$ à 1 $m^2/s$. La viscosité des silicones est par exemple mesurée à 25°C selon la norme ASTM 445 Appendice C.

Parmi ces polyalkylsiloxanes, on peut citer à titre non limitatif les produits commerciaux suivants :

- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

Dans cette classe de polyalkylsiloxanes, on peut également citer les produits commercialisés sous les dénominations "ABIL WAX 9800 et 9801" par la société GOLDSCHMIDT qui sont des polyalkyl ($C_1$-$C_{20}$) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de $1.10^{-5}$ à $5.10^{-2} m^2/s$ à 25°C.

Parmi ces polyalkylarylsiloxanes on peut citer à titre d'exemple les produits commercialisés sous les dénominations suivantes :

. les huiles SILBIONE de la série 70 641 de RHONE POULENC ;
. les huiles des séries RHODORSIL 70 633 et 763 de RHONE POULENC :

- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :

- polydiméthylsiloxane
- les gommes polydiméthylsitoxaneslméthytvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :

- les mélanges formés à partir d'un polydiméthylsiloxane hydroxylé en bout de chaîne (dénommé diméthiconol selon la nomenclature du dictionnaire CTFA) et d'un polydiniéthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6} m^2/s$: Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :

$R_2SiO_{2/2}$, $R_3SiO_{1/2}$, $RSiO_{3/2}$ et $SiO_{4/2}$ dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en $C_1$-$C_4$, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl ($C_{12}$) méthicone copolyol commercialisée par la société DOW CORNING sous la dénomination Q2 5200;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;
- des groupements ammonium quaternaires comme les produits commercialisés sous les dénominations ABILQUAT

3272 et ABILQUAT 3474 par la société GOLDSCHMIDT :

- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (IX) :

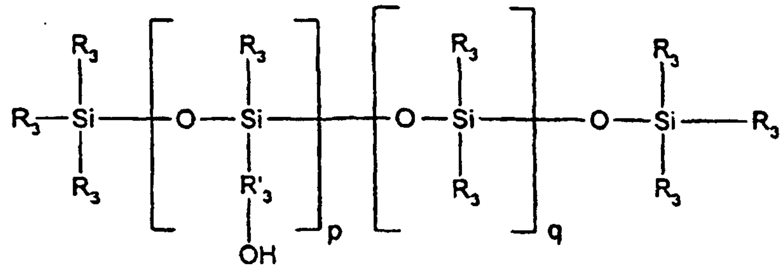

(IX)

dans laquelle les radicaux $R_3$ identiques ou différents sont choisis parmi les radicaux méthyle et phényle ; au moins 60 % en mole des radicaux $R_3$ désignant méthyle ; le radical $R'_3$ est un chaînon alkylène divalent hydrocarboné en $C_2$-$C_{18}$ ; p est compris entre 1 et 30 inclus ; q est compris entre 1 et 150 inclus ;

- des groupements acyloxyalkyle tels que par exemple les polyorganosiloxanes décrits dans le brevet US-A-4957732 et répondant à la formule (X) :

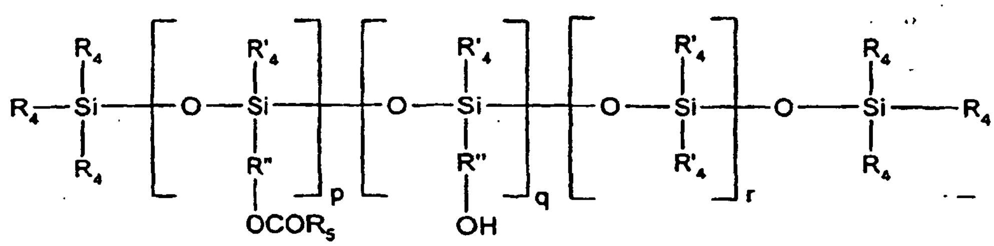

(X)

dans laquelle :

$R_4$ désigne un groupement méthyle, phényle, $-OCOR_5$, hydroxyle, un seul des radicaux $R_4$ par atome de silicium pouvant être OH ;
$R'_4$ désigne méthyle, phényle ; au moins 60 % en proportion molaire de l'ensemble des radicaux $R_4$ et $R'_4$ désignant méthyle ;
$R_5$ désigne alkyle ou alcényle en $C_8$-$C_{20}$ ;
R" désigne un radical alkylène hydrocarboné divalent, linéaire ou ramifié en $C_2$-$C_{18}$ ;
r est compris entre 1 et 120 inclus ;
p est compris entre 1 et 30 ;
q est égal à 0 ou est inférieur à 0,5 p, p + q étant compris entre 1 et 30 ; les polyorganosiloxanes de formule (VI) peuvent contenir des groupements :

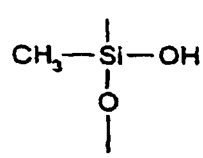

dans des proportions ne dépassant pas 15 % de la somme p + q + r.

- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".

- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :

a) 50 à 90% en poids d'acrylate de tertiobutyle ;
b) 0 à 40% en poids d'acide acrylique ;
c) 5 à 40% en poids de macromère siliconé de formule :

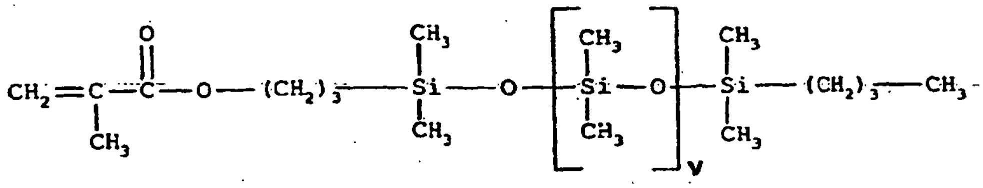

avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions, de nanoémulsions ou de micrémulsions.

Les polyorganosiloxanes particulièrement préférés conformément à l'invention sont :

- les silicones non volatiles choisies dans la famille des polyalkylsiloxanes à groupements terminaux triméthylsilyle telles que les huiles ayant une viscosité comprise entre 0,2 et 2,5 $m^2/s$ à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHONE POULENC, les polyalkylsiloxanes à groupements terminaux diméthylsilanol tels que les diméthiconol ou les polyalkylarylsiloxanes tels que l'huile SILBIONE 70641 V 200 commercialisée par la société RHONE POULENC ;
- la résine d'organopolysiloxane commercialisée sous la dénomination DOW CORNING 593 ;
- les polysiloxanes à groupements aminés tels que les amodiméthicones ou les triméthylsilylamodiméthicone ;
- les polysiloxanes à groupements ammonium quaternaires.

Selon l'invention, le ou les silicones peuvent représenter de 0,001 % à 20 % en poids, de préférence de 0,01 % à 10% en poids et plus particulièrement de 0,1 à 3% en poids par rapport au poids total de la composition finale.

Les compositions de l'invention peuvent contenir en outre au moins un agent tensioactif choisi parmi les agents tensioactifs anioniques, amphotères, non-ioniques ou leurs mélanges qui est généralement présent en une quantité comprise entre 0,1% et 60% en poids environ, de préférence entre 3% et 40% et encore plus préférentiellement entre 5% et 30%, par rapport au poids total de la composition.

Les tensioactifs convenant à la mise en oeuvre de la présente invention sont notamment les suivants :

(i) Tensioactif(s) anionique(s):

Leur nature ne revêt pas, dans le cadre de la présente invention, de caractère véritablement critique. Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl ($C_6$-$C_{24}$) éther carboxyliques polyoxyalkylénés, les acides alkyl($C_8$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

(ii) Tensioactif(s) non ionique(s):

Les agents tensioactifs non-ioniques sont, eux aussi, des composés bien connus en soi (voir notamment à cet égard "Handbook of Surfactants" par M.R. PORTER, éditions Blackie & Son (Glasgow ànd London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensioactifs non-ioniques rentrant particulièrement bien dans le cadre de la présente invention.

(iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.

Parmi les dérivés d'amines, on peut citer les produits commercialisés sous les dénomination MIRANOL, tels que décrits dans les brevets US-2 528 378 et US-2 781 354 et de structures :

$$R_2\text{-CONHCH}_2\text{CH}_2\text{ -N}(R_3)(R_4)(\text{CH}_2\text{COO-}) \qquad (2)$$

dans laquelle $R_2$ désigne un radical alkyle dérivé d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ;

et

$$R_5\text{-CONHCH}_2\text{CH}_2\text{-N(B)(C)} \qquad (3)$$

dans laquelle:

B représente $-CH_2CH_2OX'$, C représente $-(CH_2)_z$ -Y', avec z = 1 ou 2,
X' désigne le groupement $-CH_2CH_2-COOH$ ou un atome d'hydrogène
Y' désigne -COOH ou le radical $-CH_2$ - CHOH - SO3H
$R_5$ désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHONE POULENC.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl($C_{12}$-$C_{14}$) sulfates de sodium, de triéthanolamine ou d'ammonium, les alkyl ($C_{12}$-$C_{14}$)éthersulfates de sodium, de triéthanolamine ou d'ammonium oxyéthylénés à 2,2 moles d'oxyde d'éthylène, le cocoyl iséthionate de sodium et l'alphaoléfine($C_{14}$-$C_{16}$) sulfonate de sodium et leurs mélange avec :

- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

Encore plus préférentiellement les compositions selon l'invention peuvent contenir en outre au moins un tensioactif cationique.
Les tensioactifs cationiques peuvent être choisis parmi :

A) les sels d'ammonium quaternaires de la formule générale (IV) suivante :

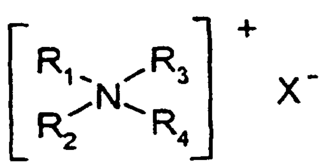

(IV)

dans laquelle X est un anion choisi dans le groupe des halogénures (chlorure, bromure ou iodure) ou alkyl($C_2$-$C_6$) sulfates plus particulièrement méthylsulfate, des phosphates, des alkyl-ou-alkylarylsulfonates, des anions dérivés d'acide organique tel que l'acétate ou le lactate,
et

i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre,

les halogènes. Les radicaux aliphatiques sont par exemple choisis parmi les radicaux alkyle, alcoxy, alkylamide,

R4 désigne un radical alkyle, linéaire ou ramifié, comportant de 16 à 30 atomes de carbone.

De préférence le tensioactif cationique est un sel (par exemple chlorure) de béhényl triméthyl ammonium.

ii) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;

R3 et R4, identiques ou différents, désignent un radical alkyle, linéaire ou ramifié, comportant de 12 à 30 atomes de carbone, ledit radical comprenant au moins une fonction ester ou amide.

R3 et R4 sont notamment choisis parmi les radicaux alkyl($C_{12}$-$C_{22}$)amido alkyle($C_2$-$C_6$), alkyl($C_{12}$-$C_{22}$) acétate ;

De préférence le tensioactif cationique est un sel (par exemple chlorure) de stéaramidopropyl diméthyl (myristylacétate) ammonium.

B) - les sels d'ammonium quaternaire de l'imidazolinium, comme par exemple celui de formule (V) suivante :

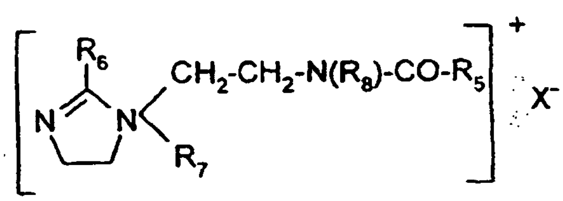

(V)

dans laquelle $R_5$ représente un radical alcényle ou alkyle comportant de 8 à 30 atomes de carbone par exemple dérivés des acides gras du suif, $R_6$ représente un atome d'hydrogène, un radical alkyle en $C_1$-$C_4$ ou un radical alcényle ou alkyle comportant de 8 à 30 atomes de carbone, $R_7$ représente un radical alkyle en $C_1$-$C_4$ , $R_8$ représente un atome d'hydrogène, un radical alkyle en $C_1$-$C_4$, X est un anion choisi dans le groupe des halogénures, phosphates, acétates, lactates, alkylsulfates, alkyl-ou-alkylarylsulfonates. De préférence, $R_5$ et $R_6$ désignent un mélange de radicaux alcényle ou alkyle comportant de 12 à 21 atomes de carbone par exemple dérivés des acides gras du suif, $R_7$ désigne méthyle, $R_8$ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,

C) - les sels de diammonium quaternaire de formule (VI) :

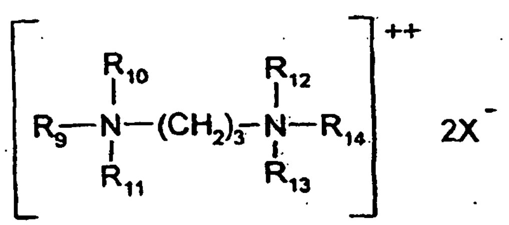

(VI)

dans laquelle $R_9$ désigne un radical aliphatique comportant environ de 16 à 30 atomes de carbone, $R_{10}$, $R_{11}$, $R_{12}$, $R_{13}$ et $R_{14}$, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.

D) - les sels d'ammonium quaternaire contenant au moins une fonction ester de formule (VII) suivante :

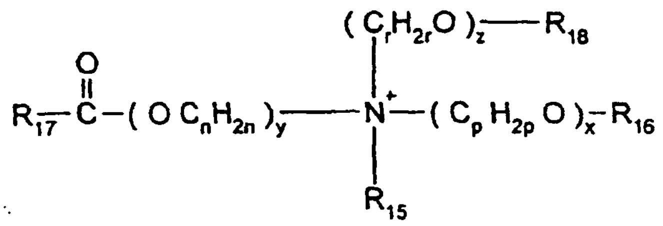 , $X^-$ (VII)

dans laquelle :

- $R_{15}$ est choisi parmi les radicaux alkyles en $C_1$-$C_6$ et les radicaux hydroxyalkyles ou dihydroxyalkyles en $C_1$-$C_6$;
- $R_{16}$ est choisi parmi :
  - le radical

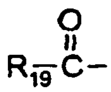

  - les radicaux $R_{20}$ hydrocarbonés en $C_1$-$C_{22}$ linéaires ou ramifiés, saturés ou insaturés,
  - l'atome d'hydrogène,
- $R_{18}$ est choisi parmi :
  - le radical

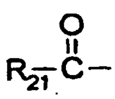

  - les radicaux $R_{22}$ hydrocarbonés en $C_1$-$C_6$ linéaires ou ramifiés, saturés ou insaturés,
  - l'atome d'hydrogène,
- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- $X^-$ est un anion simple ou complexe, organique ou inorganique ;

sous réserve que la somme x + y + z vaut de 1 à 15, que lorsque x vaut 0 alors $R_{16}$ désigne $R_{20}$ et que lorsque z vaut 0 alors $R_{18}$ désigne $R_{22}$.

On utilise plus particulièrement les sels d'ammonium de formule (VII) dans laquelle :

- $R_{15}$ désigne un radical méthyle ou éthyle,
- x et y sont égaux à 1 ;
- z est égal à 0 ou 1 ;
- n, p et r sont égaux à 2 ;
- $R_{16}$ est choisi parmi :
  - le radical

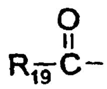

- les radicaux méthyle, éthyle ou hydrocarbonés en $C_{14}$-$C_{22}$
- l'atome d'hydrogène ;

- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- $R_{18}$ est choisi parmi :
  - le radical

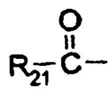

  - l'atome d'hydrogène ;

De tels composés sont par exemple commercialisés sous les dénominations DEHYQUART par la société HENKEL, STEPANQUAT par la société STEPAN, NOXAMIUM par la société CECA, REWOQUATWE 18 par la société REWO-WITCO.
Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.
Le tensioactif cationique est généralement présent dans des concentrations allant de 0,1 à 10% en poids par rapport au poids total de la composition et de préférence de 0,5 à 7 % en poids et plus préférentiellement entre 1 et 5% en poids.
La composition de l'invention peut également contenir au moins un additif choisi parmi les épaississants, les parfums, les agents nacrants, les conservateurs, les filtres solaires siliconés ou non, les vitamines, les provitamines, les polymères cationiques, amphotères, anioniques ou non ioniques, les protéines, les hydrolysats de protéïne, l'acide méthyl-18 eicosanoique, les hydroxyacides, le panthénol, les huiles végétales, animales ou minérales, les huiles de synthèse, les céramides, les pseudocéramides et tout autre additif classiquement utilisé dans le domaine cosmétique qui n'affecte pas les propriétés des compositions selon l'invention.
Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.
Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.
Les compositions selon l'invention peuvent être des compositions d'après-shampooing à rincer ou non.
Les compositions selon l'invention peuvent être également des compositions détergentes telles que des shampooings, des gels-douche, des bains moussants et peuvent être également des démaquillants. Dans ce mode de réalisation de l'invention, les compositions comprennent une base lavante, généralement aqueuse.
Le ou les tensioactifs formant la base lavante peuvent être indifféremment choisis, seuls ou en mélanges, au sein des tensioactifs anioniques, amphotères et non ioniques tels que définis ci-dessus.
La quantité et la qualité de la base lavante sont celles suffisantes pour conférer à la composition finale un pouvoir moussant et/ou détergent satisfaisant.
Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.
L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.
Ainsi, ce procédé selon l'invention permet le maintien de la coiffure, le traitement, le soin ou le lavage ou le démaquillage de la peau, des cheveux ou de toute autre matière kératinique.
Les compositions de l'invention peuvent également se présenter sous forme de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

EXEMPLE 1

On a réalisé un après-shampooing conforme à l'invention de composition suivante :

- Mélange de myristate, palmiate et stéarate de myristyle, cétyle et stéaryle 1 g
- Alcool cétylique 5 g
- Hydroxyéthylcellulose (PM 1.300.000) 0,25 g
- Chlorure de béhényl triméthyl ammonium (GENAMIN KDMP de CLARIANT) 1 g MA
- Emulsion cationique à 67% MA de copolymère polydiméthylsiloxane à groupements alpha-oméga vinyle / polydiméthylsiloxane à groupements alpha-oméga hydrogéno (DC-1997 de DOW CORNING) 0,8 gMA
- Polydiméthylsiloxane (MIRASIL DM300 de RHODIA CHIMIE) 5 g
- Polydiméthylsiloxane (DC200 FLUID-60.000CS de DOW CORNING) 1 g
- Parfum, conservateurs qs
- Eau qsp 100 g

On applique cette composition sur des cheveux lavés et essorés. On laisse pauser pendant 2 minutes, puis on rince à l'eau.
Les cheveux traités avec cet après-shampooing sont doux, lisses et se démêlent facilement.

EXEMPLE 2

On a réalisé un après-shampooing conforme à l'invention de composition suivante :

- Emulsion cationique à 67% MA de copolymère polydiméthylsiloxane à groupements alpha-oméga vinyle / polydiméthylsiloxane à groupements alpha-oméga hydrogéno (DC-1997 de DOW CORNING) 0,7 gMA
- Copolymère SMDI / polyéthylène glycol / terminaisons alkyle (méthyl/C18) à 15% dans une matrice maltodextrine 1 eau (ACULYN 46 de ROHM HAAS) 0,45 gMA
- Homopolymère chlorure de méthacrylate d'éthyl triméthyl ammonium réticulé en émulsion inverse à 50% dans de l'huile minérale (SALCARE SC 95 de CIBA GEIGY) 0,55 gMA
- Polydiméthylsiloxane (MIRASIL DM50 de RHODIA CHIMIE) 1 g
- Mélange d'alcool cétylique et d'alcool stéarylique (50/50 en poids) 6 g
- Parfum, conservateurs qs
- Eau qsp 100 g

## Revendications

1. Composition cosmétique, **caractérisée par le fait qu'**elle comprend, dans un milieu cosmétiquement acceptable, au moins une silicone et au moins un copolymère siliconé de viscosité comprise entre $10^6$ et $100.10^6$ cP résultant de la réaction d'addition, en présence d'un catalyseur, d'au moins :

- (a) un polysiloxane de formule (I) ;

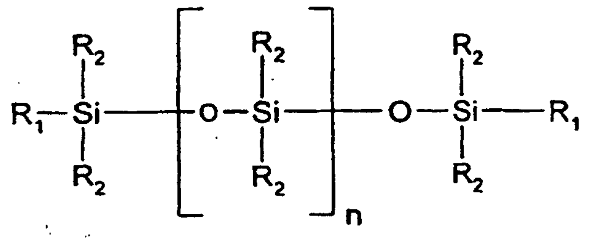

(I)

dans laquelle :
R1 désigne un groupement susceptible de réagir par réaction d'addition de chaîne tel que par exemple un atome d'hydrogène, un groupements aliphatique éthylénique monoinsaturé notamment vinyl, allyl ou héxényl, les groupements R2 de la formule (I) représentent des groupements alkyle ayant de 1 à 20 atomes de carbone, cycloalkyle ayant 5 ou 6 atomes de carbone, aryle, alkylaryle ayant de 7 à 20 atomes de carbone ou hydroxyle, et peuvent comporter en outre, des groupements fonctionnels tels qu'éthers, amines, carboxyles, hydroxyles, thiols, esters, sulfonates, sulfates,
n est un entier tel que le polysiloxane de formule (I) ait de préférence une viscosité cinématique comprise entre 1 et $1.10^6$ $mm^2/s$.

- (b) et d'au moins un composé siliconé comprenant au moins un et au plus deux groupements susceptibles de réagir avec les groupements R1 du polysiloxane (a),

l'un au moins des composés de type (a) ou (b) contient un groupe aliphatique ayant une insaturation éthylénique.

2. Composition selon la revendication 1, **caractérisée par le fait que** R2 désigne méthyle.

3. Composition selon l'une des revendications 1 ou 2, **caractérisée par le fait que** le composé de type (b) est un autre polysiloxane de type (a) dans lequel les groupements R1 du polysiloxane (b) sont susceptibles de réagir avec les groupement R1 du polysiloxane (a).

4. Composition selon l'une quelconque des revendications 1 à 3, **caractérisée par le fait que** le copolymère siliconé est obtenu par réaction d'addition, en présence d'un catalyseur d'hydrosilylation, d'au moins :

   - (a) un alpha,oméga-di vinyl polydiméthylsiloxane, et
   - (b) d'un alpha,oméga-dihydrogéno polydiméthylsiloxane.

5. Composition selon l'une quelconque des revendications 1 à 4, **caractérisée par le fait que** le copolymère siliconé est sous la forme d'émulsion aqueuse.

6. Composition selon l'une quelconque des revendications 1 à 5, **caractérisée par le fait que** le copolymère siliconé est présent à une concentration comprise entre 0,05 et 10% en poids par rapport au poids total de la composition.

7. Composition selon l'une quelconque des revendications 1 à 6, **caractérisée par le fait que** les silicones différentes du copolymère siliconé sont des potyorganosiloxanes choisis parmi les polyalkylsiloxanes, les polyarylsiloxanes, les polyalkylarylsiloxanes, les gommes et résines de silicones, les polyorganosiloxanes modifiés par des groupements organofonctionnels ainsi que leurs mélanges.

8. Composition selon la revendication 7, **caractérisée par le fait que** :

   (a) les polyalkylsiloxanes sont choisis parmi :

   - les polydiméthylsiloxanes à groupements terminaux triméthylsilyle :

- les polydiméthylsiloxanes à groupements terminaux diméthylsilanol ;
- les polyalkyl($C_1$-$C_{20}$)siloxanes;

(b) les polyalkylarylsiloxanes sont choisis parmi :

- les polydimèthylméthylphénylsiloxanes, les polydiméthyldiphényl siloxanes linéaires et/ou ramifiés de viscosité comprise entre 1. $10^{-5}$ et $5.10^{-2} m^2/s$ à 25°C ;

(c) les gommes de silicone sont choisies parmi les polydiorganosiloxanes ayant des masses moléculaires moyennes en nombre comprises entre 200 000 et 1 000 000 utilisés seuls ou sous forme de mélange dans un solvant ;
(d) les résines sont choisies parmi les résines constituées d'unités : $R_3$ Si $O_{1/2}$, $R_2$ Si $O_{2/2}$, R Si $O_{3/2}$, Si $O_{4/2}$ dans lesquelles R représente un groupement hydrocarboné ayant de 1 à 16 atomes de carbone ou un groupement phényle ;
(e) les silicones organo modifiées sont choisies parmi les silicones comportant dans leur structure un ou plusieurs groupements organofonctionnels fixés par l'intermédiaire d'un radical hydrocarboné.

**9.** Composition selon l'une quelconque des revendications 7 ou 8, **caractérisée par le fait que** les gommes de silicone utilisées seules ou sous forme de mélange sont choisies parmi les structures suivantes :

- polydiméthylsiloxane
- polydiméthylsiloxane/méthylvinylsiloxanes,
- polydiméthylsiloxane/diphénylsiloxane,
- polydiméthylsiloxane/phénylméthylsiloxane,
- polydiméthylsiloxane/diphénylsitoxane/méthylvinylsiloxanes et les mélanges suivants :
- des mélanges formés à partir d'un polydiméthylsiloxane hydroxylé en bout de chaîne et d'un polydiméthylsiloxane cyclique ;
- lés mélanges formés à partir d'une gomme polydiméthylsiloxane et d'une silicone cyclique ; et
- des mélanges de polydiméthylsiloxanes de viscosités différentes.

**10.** Composition selon l'une quelconque des revendications 7 ou 8, **caractérisée par le fait que** les silicones organomodifiées sont choisies parmi les polyorganosiloxanes comportant :

a) des groupements polyéthylèneoxy et/ou polypropylèneoxy ;
b) des groupements aminés substitués ou non ;
c) des groupements thiols ;
d) des groupements alcoxylés,
e) des groupements hydroxyalkyle,
f) des groupements acyloxyalkyle,
g) des groupements alkyl carboxyliques,
h) des groupements 2-hydroxyalkylsulfonates,
i) des groupements 2-hydroxyalkylthiosulfonates,
j) des groupements hydroxyacylamino,
k) des groupements ammonium quaternaires.

**11.** Composition selon l'une quelconque des revendications 7 à 10, **caractérisée par le fait que** les polyorganosiloxanes sont choisis parmi les polyalkylsiloxanes à groupements terminaux triméthylsilyle, les polyalkylsiloxanes à groupements terminaux diméthylsilanol, les polyalkylarylsiloxanes, les mélanges de deux PDMS constitués d'une gomme et d'une huile de viscosités différentes, les mélanges d'organosiloxanes et de silicones cycliques, les résines d'organopolysiloxanes, les polysiloxanes à groupements, les polysiloxanes à groupements ammonium quaternaires.

**12.** Composition selon l'une quelconque des revendications 1 à 11, **caractérisée par le fait que** les silicones sont choisies parmi les polyorganosiloxanes insolubles dans la composition.

**13.** Composition selon l'une quelconque des revendications précédentes, **caractérisée en ce que** la silicone est présent à une concentration comprise entre 0,001 % et 20 % en poids par rapport au poids total de la composition, de préférence entre 0,01 % et 10 % en poids.

**14.** Composition selon une quelconque des revendications précédentes, **caractérisée par le fait qu'**elle comprend en outre au moins un agent tensioactif choisi parmi les tensioactifs anioniques, non ioniques, amphotères et leurs mélanges.

**15.** Composition selon la revendication 14, **caractérisée par le fait que** le ou les agents tensioactifs sont présents à une concentration comprise entre 0,1% et 60% en poids, de préférence entre 3% et 40% en poids, et encore plus préférentiellement entre 5% et 30% en poids, par rapport au poids total de la composition.

**16.** Composition selon l'une quelconque des revendications précédentes, **caractérisée par le fait qu'**elle comprend en outre au moins un agent tensioactif cationique.

**17.** Composition selon la revendication précédente, **caractérisée par le fait que** le tensioactif cationique est présent dans des concentrations allant de 0,1 à 10% en poids par rapport au poids total de la composition et de préférence de 0,5 à 7 % en poids et plus préférentiellement entre 1 et 5% en poids.

**18.** Composition selon l'une quelconque des revendications précédentes, **caractérisée par le fait qu'**elle se présente sous forme de shampooing, d'après-shampooing à rincer ou non, de composition pour la permanente, le défrisage, la coloration ou la décoloration des cheveux, de composition à rincer à appliquer entre les deux étapes d'une permanente ou d'un défrisage, de composition lavantes pour le corps.

**19.** Utilisation d'une composition telle que définie dans l'une quelconque des revendications précédentes pour le lavage ou pour le soin des matières kératiniques.

**20.** Procédé de traitement des matières kératiniques, telles que les cheveux, **caractérisé en ce qu'**il consiste à appliquer sur lesdites matières une composition cosmétique selon l'une des revendications 1 à 18, puis à effectuer éventuellement un rinçage à l'eau.

**21.** Utilisation d'un copolymère siliconé tel que défini à l'une des revendications 1 à 5 dans, ou pour la fabrication d'une composition cosmétique comprenant une silicone.

**Patentansprüche**

**1.** Kosmetische Zusammensetzung, **dadurch gekennzeichnet, dass** sie in einem kosmetisch akzeptablen Medium mindestens ein Silicon und mindestens ein Siliconcopolymer mit einer Viskosität von $10^6$ bis $100 \cdot 10^6$ cP enthält, entstehend in Gegenwart eines Katalysators bei der Addition von zumindest:

(a) einem Polysiloxan der Formel (I):

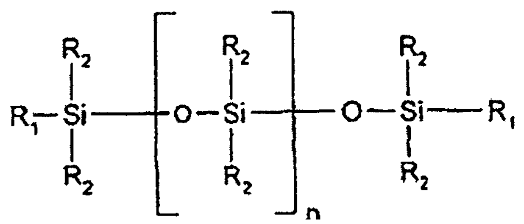
(I)

worin bedeuten:

R1 eine Gruppe, die durch Kettenaddition reagieren kann, wie beispielsweise ein Wasserstoffatom, eine aliphatische ethylenisch ungesättigte Gruppe, besonders Vinyl, Allyl oder Hexenyl,

wobei die Gruppen R2 der Formel (I) Alkylgruppen mit 1 bis 20 Kohlenstoffatomen, Cycloalkylgruppen mit 5 oder 6 Kohlenstoffatomen, Arylgruppen, Alkylarylgruppen mit 7 bis 20 Kohlenstoffatomen oder Hydroxy bedeuten und ferner funktionelle Gruppen enthalten können, wie beispielsweise Ether, Amine, Carboxygruppen, Hydroxygruppen, Thiole, Ester, Sulfonate, Sulfate, n eine ganze Zahl, die so gewählt ist, dass das Polysiloxan der Formel (I) vorzugsweise eine kinematische Viskosität von 1 bis $1.10^6$ $mm^2/s$ aufweist;

(b) und mindestens einer Silicon-haltigen Verbindung, die mindestens eine und höchstens zwei Gruppen aufweist, die mit den Gruppen R1 des Polysiloxans (a) reagieren können,

wobei mindestens eine der Verbindungen vom Typ (a) oder (b) eine aliphatische Gruppe enthält, die ethylenisch ungesättigt ist.

**2.** Zusammensetzung nach Anspruch 1, **dadurch gekennzeichnet, dass** R2 Methyl bedeutet.

**3.** Zusammensetzung nach einem der Ansprüche 1 oder 2, **dadurch gekennzeichnet, dass** die Verbindung vom Typ (b) ein anderes Polysiloxan vom Typ (a) ist, wobei die Gruppen R1 des Polysiloxans (b) mit den Gruppen R1 des Polysiloxans (a) reagieren können.

**4.** Zusammensetzung nach einem der Ansprüche 1 bis 3, **dadurch gekennzeichnet, dass** das Siliconcopolymer in Gegenwart eines Hydrosilylierungskatalysators durch Addition hergestellt wird von zumindest:

(a) einem alpha, omega-Divinylpolydimethylsiloxan und
(b) einem alpha, omega-Dihydrogenopolydimethylsiloxan.

**5.** Zusammensetzung nach einem der Ansprüche 1 bis 4, **dadurch gekennzeichnet, dass** das Siliconcopolymer in Form einer wässerigen Emulsion vorliegt.

**6.** Zusammensetzung nach einem der Ansprüche 1 bis 5, **dadurch gekennzeichnet, dass** das Siliconcopolymer in einer Konzentration von 0,05 bis 10 Gew.-%, bezogen auf däs Gesamtgewicht der Zusammensetzung, enthalten ist.

**7.** Zusammensetzung nach einem der Ansprüche 1 bis 6, **dadurch gekennzeichnet, dass** die Silicone, die von dem Siliconcopolymer verschieden sind, Polyorganosiloxane sind, die unter den Polyalkylsiloxanen, Polyarylsiloxanen, Polyalkylarylsiloxanen, Silicongummis und Siliconharzen, mit organofunktionellen Gruppen modifizierten Polyorganosiloxanen sowie deren Gemischen ausgewählt sind.

**8.** Zusammensetzung nach Anspruch 7, **dadurch gekennzeichnet, dass**

(a) die Polyalkylsiloxane ausgewählt sind unter:

- Polydimethylsiloxanen mit endständigen Trimethylsilylgruppen;
- Polydimethylsiloxanen mit endständigen Dimethylsilanolgruppen;
- Polyalkyl($C_{1-20}$)siloxanen;

(b) die Polyalkylarylsiloxane ausgewählt sind unter:

- Polydimethylmethylphenylsiloxanen, Polydimethyldiphenylsiloxanen, die geradkettig und/oder verzweigt vorliegen und bei 25 °C eine Viskosität im Bereich von $1 \cdot 10^{-5}$ bis $5.10^{-2}$ $m^2/s$ besitzen;

(c) die Silicongummis unter den Polydiorganosiloxanen ausgewählt sind, die eine zahlenmittlere Molmasse von 200.000 bis 1.000.000 aufweisen, und alleine oder in Form eines Gemisches in einem Lösungsmittel verwendet werden;
(d) die Harze unter den Harzen ausgewählt sind, die aus den folgenden Einheiten bestehen: $R_3SiO_{1/2}$, $R_2SiO_{2/2}$, $RSiO_{3/2}$, $SiO_{4/2}$,
wobei R eine Kohlenwasserstoffgruppe mit 1 bis 16 Kohlenstoffatomen oder eine Phenylgruppe ist; und
(e) die organomodifizierten Silicone unter den Siliconen ausgewählt sind, die in ihrer Struktur eine oder mehrere organofunktionelle Gruppen aufweisen, die über eine Kohlenwasserstoffgruppe gebunden sind.

**9.** Zusammensetzung nach einem der Ansprüche 7 oder 8, **dadurch gekennzeichnet, dass** die Silicongummis, die einzeln oder in Form eines Gemisches verwendet werden, unter den folgenden Strukturen ausgewählt sind:

- Polydimethylsiloxan

- Polydimethylsiloxan/ Methylvinylsiloxane,
- Polydimethylsiloxan/Diphenylsiloxan,
- Polydimethylsiloxan/ Phenylmethylsiloxan,
- Polydimethylsiloxan/Diphenylsiloxan/Methylvinylsiloxane und den folgenden Gemischen:
- Gemischen, die aus einem am Kettenende hydroxylierten Polydimethylsiloxan und einem cyclischen Polydimethylsiloxan gebildet sind,
- Gemischen, die aus einem Polydimethylsiloxangummi und einem cyclischen Silicon gebildet werden; und
- Gemischen von Polydimethylsiloxanen unterschiedlicher Viskositäten.

**10.** Zusammensetzung nach einem der Ansprüche 7 oder 8, **dadurch gekennzeichnet, dass** die organomodifizierten Silicone unter den Polyorganosiloxanen ausgewählt sind, die enthalten:

a) Polyethylenoxy- und/oder Polypropylenoxygruppen;
b) substituierte oder unsubstituierte aminierte Gruppen;
c) Thiolgruppen;
d) alkoxylierte Gruppen,
e) Hydroxyalkylgruppen,
f) Acyloxyalkylgruppen,
g) Alkylcarboxygruppen,
h) 2-Hydroxyalkylsulfonatgruppen,
i) 2-Hydroxyalkylthiosulfonatgruppen,
j) Hydroxyacylaminogruppen,
k) quartäre Ammoniumgruppen.

**11.** Zusammensetzung nach einem der Ansprüche 7 bis 10, **dadurch gekennzeichnet, dass** die Polyorganosiloxane unter den Polyalkylsiloxanen mit endständigen Trimethylsilylgruppen, Polyalkylsiloxanen mit endständigen Dimethylsilanolgruppen, Polyalkylarylsiloxanen, Gemischen von zwei PDMS, die aus einem Gummi und einem Öl unterschiedlicher Viskositäten bestehen, Gemischen von Organosiloxanen und cyclischen Siliconen, Organopolysiloxanharzen, Polysiloxanen mit Amino Gruppen und Polysiloxanen mit quartären Ammoniumgruppen ausgewählt sind.

**12.** Zusammensetzung nach einem der Ansprüche 1 bis 11, **dadurch gekennzeichnet, dass** die Silicone unter den in der Zusammensetzung unlöslichen Polyorganosiloxanen ausgewählt sind.

**13.** Zusammensetzung nach einem der vorhergehenden Ansprüche, **dadurch gekennzeichnet, dass** das Silicon in einer Konzentration von 0,01 bis 20 Gew.-%, bezogen auf das Gesamtgewicht der Zusammensetzung, und vorzugsweise 0,01 bis 10 Gew.-%, enthalten ist.

**14.** Zusammensetzung nach einem der vorhergehenden Ansprüche, **dadurch gekennzeichnet, dass** sie ferner mindestens einen grenzflächenaktiven Stoff enthält, der unter den anionischen, nichtionischen oder amphoteren grenzflächenaktiven Stoffen und deren Gemischen ausgewählt ist.

**15.** Zusammensetzung nach Anspruch 14, **dadurch gekennzeichnet, dass** der oder die grenzflächenaktive(n) Stoff (e) in einer Konzentration von 0,1 bis 60 Gew.-%, vorzugsweise 3 bis 40 Gew.-% und noch bevorzugter 5 bis 30 Gew.-%, bezogen auf das Gesamtgewicht der Zusammensetzung, enthalten sind.

**16.** Zusammensetzung nach einem der vorhergehenden Ansprüche, **dadurch gekennzeichnet, dass** sie ferner mindestens einen kationischen grenzflächenaktiven Stoff enthält.

**17.** Zusammensetzung nach dem vorhergehenden Anspruch, **dadurch gekennzeichnet, dass** der kationische grenzflächenaktive Stoff in Konzentrationen von 0,1 bis 10 Gew.-%, bezogen auf das Gesamtgewicht der Zusammensetzung, und vorzugsweise 0,5 bis 7 Gew.-% und noch bevorzugter 1 bis 5 Gew.-% enthalten ist.

**18.** Zusammensetzung nach einem der vorhergehenden Ansprüche, **dadurch gekennzeichnet, dass** sie als Haarwaschmittel, Conditioner, der ausgespült wird oder im Haar verbleibt, Zusammensetzung für dauerhafte Verformungen, Entkräuselungen, Färbungen oder Entfärbungen der Haare, Zusammensetzung, die ausgespült wird und zwischen den beiden Schritten einer permanenten Verformung oder Entkräuselung aufgebracht wird oder reinigende Zusammensetzung für den Körper vorliegt.

**19.** Verwendung einer Zusammensetzung nach einem der vorhergehenden Ansprüche zur Reinigung oder zur Pflege von Keratinsubstanzen.

**20.** Verfahren zur Behandlung von Keratinsubstanzen, beispielsweise zur Behandlung der Haare, das **dadurch gekennzeichnet ist, dass** es darin besteht, auf diese Substanzen eine kosmetische Zusammensetzung nach einem der Ansprüche 1 bis 18 aufzutragen und gegebenenfalls mit Wasser zu spülen.

**21.** Verwendung eines Siliconcopolymers nach einem der Ansprüche 1 bis 5 in einer kosmetischen Zusammensetzung, die ein Silicon enthält, oder zur Herstellung einer solchen Zusammensetzung.

## Claims

**1.** Cosmetic composition, **characterized in that** it comprises, in a cosmetically acceptable medium, at least one silicone and at least one silicone copolymer with a viscosity of between $10^6$ and $100 \times 10^6$ cP, resulting from the addition reaction, in the presence of a catalyst, of at least:

- (a) one polysiloxane of formula (I) :

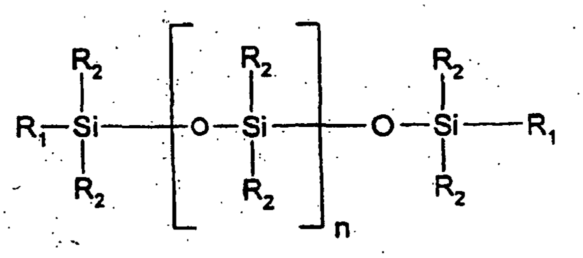

(I)

in which:

$R_1$ denotes a group which can react by chain addition reaction such as, for example, a hydrogen atom or an aliphatic group containing an ethylenic unsaturation, in particular vinyl, allyl or hexenyl;

the groups $R_2$ in formula (I) represent alkyl groups containing from 1 to 20 carbon atoms, cycloalkyl groups containing from 5 to 6 carbon atoms, aryl groups, alkylaryl groups containing from 7 to 20 carbon atoms or hydroxyl groups and can also comprise functional groups such as ethers, amines, carboxyls, hydroxyls, thiols, esters, sulphonates or sulphates.

n is an integer such that the polysiloxane of formula (I) preferably has a kinetic viscosity of between 1 and $1 \times 10^6$ $mm^2/s$ .

- (b) and of at least one silicone compound comprising at least one and not more than two groups capable of reacting with the groups $R_1$ of the polysiloxane (a), at least one of the compounds of type (a) or (b) contains an aliphatic group containing an ethylenic unsaturation.

**2.** Composition according to Claim 1, **characterized in that** $R_2$ denotes methyl.

**3.** Composition according to either of Claims 1 and 2, **characterized in that** the compound of type (b) is another polysiloxane of type (a) in which the groups $R_1$ of the polysiloxane (b) can react with the groups $R_1$ of the polysiloxane (a).

**4.** Composition according to any one of Claims 1 to 3, **characterized in that** the silicone copolymer is obtained by addition reaction, in the presence of a hydrosilylation catalyst, of at least:

- (a) one $\alpha,\omega$-divinylpolydimethylsiloxane, and
- (b) one $\alpha,\omega$-dihydrogenopolydimethylsiloxane.

**5.** Composition according to any one of Claims 1 to 4, **characterized in that** the silicone copolymer is in the form of an aqueous emulsion.

6. Composition according to any one of Claims 1 to 5, **characterized in that** the silicone copolymer is present at a concentration of between 0.05% and 10% by weight relative to the total weight of the composition.

7. Composition according to any one of Claims 1 to 6, **characterized in that** the silicones other than the silicone copolymer are polyorganosiloxanes chosen from polyalkylsiloxanes, polyarylsiloxanes, polyalkylaryl-siloxanes, silicone gums and resins and polyorgano-siloxanes modified with organofunctional groups, as well as mixtures thereof.

8. Composition according to Claim 7, **characterized in that**:

(a) the polyalkylsiloxanes are chosen from:

- polydimethylsiloxanes containing trimethylsilyl end groups;
- polydimethylsiloxanes containing dimethylsilanol end groups;
- poly ($C_1$-$C_{20}$) alkylsiloxanes;

(b) the polyalkylarylsiloxanes are chosen from:

- polydimethylmethylphenylsiloxanes, linear and/or branched polydimethyldiphenylsiloxanes with a viscosity of between $1 \times 10^{-5}$ and $5 \times 10^{-2}$ $m^2/s$ at 25°C;

(c) the silicone gums are chosen from polydiorgano-siloxanes with number-average molecular masses of between 200,000 and 1,000,000, used alone or in the form of a mixture in a solvent;
(d) the resins are chosen from resins consisting of units: $R_3SiO_{1/2}$,$R_2SiO_{2/2}$,$RSiO_{3/2}$,$SiO_{4/2}$
in which R represents a hydrocarbon-based group containing from 1 to 16 carbon atoms or a phenyl group;
(e) the organomodified silicones are chosen from silicones comprising in their structure one or more organofunctional groups attached via a hydrocarbon-based radical.

9. Composition according to either of Claims 7 and 8, **characterized in that** the silicone gums used, alone or in the form of a mixture, are chosen from the following structures:

- polydimethylsiloxane,
- polydimethylsiloxane/methylvinylsiloxanes,
- polydimethylsiloxane/diphenylsiloxane,
- polydimethylsiloxane/phenylmethylsiloxane;
- polydimethylsiloxane/diphenylsiloxane/methylvinyl-siloxanes and the following mixtures:
- mixtures formed from a polydimethylsiloxane which is hydroxylated at the end of the chain and from a cyclic polydimethylsiloxane;
- mixtures formed from a polydimethylsiloxane gum and from a cyclic silicone; and
- mixtures of polydimethylsiloxanes of different viscosities.

10. Composition according to either of Claims 7 and 8, **characterized in that** the organo-modified silicones are chosen from polyorganosiloxanes comprising:

a) polyethylenoxy and/or polypropylenoxy groups;
b) substituted or unsubstituted amine groups;
c) thiol groups;
d) alkoxylated groups,
e) hydroxyalkyl groups,
f) acyloxyalkyl groups,
g) alkylcarboxylic groups,
h) 2-hydroxyalkylsulphonate groups,
i) 2-hydroxyalkylthiosulphonate groups,
j) hydroxyacylamino groups,
k) quaternary ammonium groups.

11. Composition according to any one of Claims 7 to 10, **characterized in that** the polyorgano-siloxanes are chosen from polyalkylsiloxanes containing trimethylsilyl end groups, polyalkylsiloxanes containing dimethylsilanol end

groups, polyalkylaryl-siloxanes, mixtures of two PDMSs consisting of a gum and an oil of different viscosities, mixtures of organosiloxanes and of cyclic silicones, polyorgano-siloxane resins, polysiloxanes containing amine groups and polysiloxanes containing quaternary ammonium groups.

**12.** Composition according to any one of Claims 1 to 11, **characterized in that** the silicones are chosen from polyorganosiloxanes that are insoluble in the composition.

**13.** Composition according to any one of the preceding claims, **characterized in that** the silicone is present at a concentration of between 0.001% and 20% by weight relative to the total weight of the composition, preferably between 0.01% and 10% by weight.

**14.** Composition according to any one of the preceding claims, **characterized in that** it also comprises at least one surfactant chosen from anionic, nonionic and amphoteric surfactants, and mixtures thereof.

**15.** Composition according to Claim 14, **characterized in that** the surfactant(s) is (are) present at a concentration of between 0.1% and 60% by weight, preferably between 3% and 40% by weight and even more preferably between 5% and 30% by weight, relative to the total weight of the composition.

**16.** Composition according to any one of the preceding claims, **characterized in that** it also comprises at least one cationic surfactant.

**17.** Composition according to the preceding claim, **characterized in that** the cationic surfactant is present in concentrations ranging from 0.1% to 10% by weight relative to the total weight of the composition, and preferably from 0.5% to 7% by weight and more preferably between 1% and 5% by weight.

**18.** Composition according to any one of the preceding claims, **characterized in that** it is in the form of a shampoo, a rinse-but or leave-in conditioner, a composition for permanent-waving, straightening, dyeing or bleaching the hair, a rinse-out composition to be applied between the two steps of a permanent-waving or hair-straightening operation, or washing compositions for the body.

**19.** Use of a composition as defined in any one of the preceding claims, for washing or caring for keratin materials.

**20.** Process for treating keratin materials, such as the hair; **characterized in that** it consists in applying a cosmetic composition according to one of Claims 1 to 18, to the said keratin materials, and then in optionally rinsing it out with water.

**21.** Use of a silicone copolymer as defined in one of Claims 1 to 5, in, or for the manufacture of, a cosmetic composition comprising a silicone.